# EUROPEAN PATENT APPLICATION

(11) **EP 1 136 067 A1**
(43) Date of publication of application: **26.09.2001**
(21) Application number: 01302021.9
(22) Date of filing: 06.03.2001
(51) Int. Cl.: A61K 9/00, A23K 1/18, A23G 3/30, A61K 7/16

(54) **Oral formulations of medicaments**

(30) Priority: 23.03.2000 GB 0007112; 03.05.2000 GB 0010846
(71) Applicant: Pfizer Limited, Sandwich, Kent CT13 9NJ (GB); PFIZER INC., New York, N.Y. 10017 (US)
(72) Inventor: Huatan, Hiep, Pfizer Central Research, Sandwich, Kent CT13 9NJ (GB); Nock, Alison Jane Soards, Pfizer Central Research, Sandwich, Kent CT13 9NJ (GB); Papadopoulos, Dimitris, Pfizer Central Research, Sandwich, Kent CT13 9NJ (GB)
(74) Representative: Motion, Keith Robert

(57) **Abstract**

A formulation suitable for oral treatment with a medicament comprising:
a solid masticable portion and one or more reservoir portions encompassed by said masticable portion;
the solid masticable portion consisting of a fully edible material, having mechanical properties conducive to promoting mastication, typified by a stiffness measurement (i.e. Young's modulus) in the range of 0.01-5MPa, and compressive strength in the range of 10 to 10,000 mJ;
the reservoir portion or portions comprising a releasable unit dose of the medicament in a fluid (preferably a liquid) form, with a viscosity below 800mPa.s at body temperature (ca. 36-ca.40°C), and preferably between the range of 50-200mPa.s;
such that on mastication, the masticable portion is ruptured and the unit dose of the medicament is released in a short space of time from the reservoir portion into the oral cavity.

## Description

This invention relates to new oral formulations of medicaments, especially in relation to oral health care, especially suitable for the treatment of animals, especially companion animals such as dogs and cats.

The formulations may also be useful in the treatment of certain human subjects, e.g. those who may have difficulty in maintaining oral hygiene via conventional methods e.g. via oral brushing.

Administration of a medicament to the oral cavity of animals, particularly via gels, paste, solution etc., especially for the purpose of oral health, is recognized to be difficult and inconvenient due to the need to maintain the animal's mouth open involuntarily for a prolonged period, to enable dosing. The problem may also be encountered in the human subject groups mentioned above.

Approaches to overcome this drawback relate principally to the use of mechanically robust chewable dosage forms including rawhide, dried foodstuff, natural and synthetic polymers impregnated with medicament, which can be offered to the animal for consumption, negating the need for invasive manipulation within the animal's mouth.

For example EP 0 272 968 and WO 94/05252 disclose chewable rawhide and dried foodstuff such as beef tendon or ligament which are impregnated with medicament, for example: sodium fluoride (anti-caries agent), sodium benzoate (anti-calculus agent) and bromochlorophene (antimicrobial agent), for use as oral health products for dogs and other domestic animals.

US 4,892,748 and EP 0 552 897 B1 disclose degradable chew matrices based on cellulosic fibrous material bound with a gum system such as methyl cellulose, to impart flexibility, wherein the matrices may be incorporated with additives such as inorganic pyrophosphate salts to control tartar.

US 5,683,722 discloses a dosage form for orally administering chemical or medicinal substances such as vitamins, trace elements, amino acids, nutritive substances etc., to domestic or wild animals, wherein the dosage form comprises a palatable hydrophobic outer shell and a porous water-soluble core impregnated with the drug.

In the above disclosed systems, the release of medicament occurs by a process of dissolution from the matrix as a result of prolonged and constant gnawing or chewing on the dosage form to facilitate exposure in the oral cavity. Exposure of the medicament within the oral cavity is recognized to be discontinuous and variable as the animals do not generally gnaw or chew on the entire dosage form over a continuous interval to fully extract the medicament. The efficiency of dose delivery is therefore substantially imprecise and erratic, which can lead to poor therapeutic control and the potential for resistance development particularly with antimicrobial and anti-fungal therapies.

Unit dose chewing gum-based systems, as exemplified by US-5,498,429, US-5,736,175 and US-4,564,519 for human use, wherein the entire dosage form may be consumed within the oral cavity over a continuous interval, and wherein the chewed gum is later discarded, can overcome the discontinuity in release. Such systems however are not suitable for animal use since the gum base cannot easily be voluntarily discarded, potentially giving rise to gastrointestinal obstruction via ingestion thereof. In addition, the chewing action of the animal cannot be voluntarily controlled as in the normal human situation to ensure rapid and complete release of the medicament from the dosage form.

Liquid-filled dosage forms are available commercially for human use, and are known as Lockets®, Halls®, etc., wherein the medicament, typically menthol, is contained in the liquid-filled centre encased by a stiff and brittle outer shell. The stiff and brittle nature of the outer shell renders it difficult to chew. If chewed, the fracture is often rapid, referred to as catastrophic (i.e., giving rise to rapid propagation and branching), to release the medicament, which is then swallowed. The medicament is thus designed to be released and act at or near the nasal-oesophageal opening to relieve sore throats and aid nasal congestion. The fracturing process also gives rise to smaller fragments that can be swallowed directly or may require very limited mastication prior to swallowing. Whilst the release of medicament is rapid, the brittle nature of these systems do not lend themselves to prolonged mastication to enable the distribution of the medicament in the oral cavity to facilitate good local oral exposure. The typical compressive Young's modulus values of the shell used in these systems are of the order of 20MPa, which is high and typically requires significant force to incur fracture.

US-4,428,927 discloses a masticatory soft elastic gelatine capsule, also based on the liquid-filled concept, for the delivery of medicaments orally. The masticatory substance, as described in this invention however, is typically very soft and based on a non-digestible gum material, designed to promote rapid rupture and release of the inner fill, leaving just the outer shell, which can then be discarded.
In alternative embodiments as for example described in WO00/51574 and US-6,027,746, the components of the outer shell is specifically modified to be edible and therefore overcome the problem of ingestion. The mechanical properties of these systems however, are described as "soft", and are designed to be, at least, partially dispersed or dissolved in the mouth over a very short period (<60sec) following administration. The rapid dissolution would thus result in a significant loss of mechanical integrity, which would discourage or inhibit continued mastication to promote good local distribution of the encapsulated material within the oral cavity.

WO00/01372 describes a digestible oral dosage form, wherein the encapsulated active substrate is presented in multiple reservoirs. The release profile of the substrate, according to this disclosure, is gradual and is controlled by the degree of chewing. Hence in application to animals wherein the chewing action is not voluntary and could be limited in time, the expected exposure would be low, variable and at best incomplete.

Other prior art includes that disclosing human chewable medicated and confectionery products, such as Nicorette®, which contains nicotine, used to control smoking-dependency and Airwaves®, which contains menthol, used to freshen breath and clear the nasal passages. The medicaments in these systems are either dispersed in the matrix of the base (Nicorette®) or are coated onto pellets, which are then compressed into the final configuration (Airwaves®). The underlying material in both these systems is a gum base composite that is non-edible and thus is unsuitable for non-human animal use as the dosage form cannot be discarded as in the example described above.

There thus exists a need for an edible dosage form which enables more efficient, accurate and timely release of a unit dose of a medicament within the oral cavity, especially in relation to the subject groups mentioned above, and especially in relation to oral health medicaments, especially those suitable for animal treatments.

"Edible" means can be ingested without causing substantial subsequent gastrointestinal obstruction, discomfort or malfunction, preferably wholly digestible.

According to the invention, there is provided a formulation for a medicament comprising:
a solid masticable portion and one or more reservoir portions encompassed by said masticable portion;
the solid masticable portion consisting of a fully edible material, having mechanical properties conducive to promoting mastication, typified by a stiffness measurement (i.e. Young's modulus) in the range of 0.01-5MPa, and compressive strength in the range of 10 to 10,000 mJ;
the reservoir portion or portions comprising a releasable unit dose of the medicament in a fluid (preferably a liquid) form, with a viscosity below 800mPa.s at body temperature (ca. 36-ca.40°C), and preferably between the range of 50-200mPa.s;
such that on mastication, the masticable portion is ruptured and the unit dose of the medicament is released in a short space of time from the reservoir portion into the oral cavity.

The solid masticable portion consists of materials that are non-brittle, fully edible, which exhibit mechanical properties specifically tailored to encourage mastication in the oral cavity. The mechanical properties of the masticable portion can be described by two main parameters, i.e. the compressive Young's modulus and the compressive strength.

The compressive Young's modulus (E) is taken to reflect resistance to elastic deformation and may be expressed as the ratio of applied compressive stress to compressive strain (Ashby and Jones, 1980). This property is used here to quantify the chewability characteristics. For the formulations of this invention, the value for E is typically in the range of 0.01-5MPa and more preferably between the ranges of 0.1-2MPa. Measurement of this parameter is performed by compressing a cylindrical metallic plunger, 15.84mm (outer diameter), against the chew at a constant rate of 5 mm.min⁻¹ until a load of 5N is reached.

The compressive strength is taken to reflect the material's resistance to rupture and is defined as the work (energy) required to compress a sample to *ca*. 100% deformation. More specifically and similar to the Young's modulus measurement, the chew is rested on a solid metal block. Initially the plunger makes contact with the chew at 0.5 N, followed by compression at a constant rate (5 mm.min⁻¹) until a maximum force of 200 N is reached. Extensive observations have shown that this force level is sufficient to compress all chews to very high deformation levels. The compressive strength is equal to the area under the force-displacement curve, and it is calculated automatically using instrument software (Lloyd LR30K, Windows Rcontrol 1.30).
Other stress-strain relationships, as derived from typical compression tests (for example, creep), or mechanical/textural tests such as Texture Profile Analysis (Jones *et al*., 1996), Dynamic Mechanical Analysis (Jones, 1999) and/or rheological analysis may also be employed to characterise the chewability performance of the said system.

Examples of suitable masticable portion materials include edible sugars (i.e., polysaccharides) such as sucrose, glucose, dextrose, mannose, maltose, etc., and/or polyols (i.e., hydrogenated polysaccharides) such as xylitol, mannitol, sorbitol, maltitol, maltotritol, etc., as the base with viscoelastic polymer additives such as starch, gelatin, gum arabic, xantham gum and cellulosic fibres to impart elasticity and strength.

A polysaccharide and/or hydrogenated polysaccharide base is typically the dominant component of the masticable portion and will make up typically greater than about 50% of the total solid (TS) content by weight, and preferably greater than about 60% w/w, for example about 74% w/w.

The viscoelastic polymer additives such as gelatin and/or gum arabic, will make up less than about 50% of the TS content of the masticable portion by weight, and preferably less than about 30%, for example about 14%.

The masticable portion may also include mucoadhesive additives to promote retention within the oral cavity, such as acrylic-based polymers and cellulose-based polymers for example, hydroxy propyl methyl cellulose (methocel) and sodium carboxy methyl cellulose (Carbopol™). When present, these agents will make up typically less than 20% of the TS content of the masticable portion by weight.

Flavourant and colourant may also be included in the masticable portion, and when present will make up less than 30% of the total solid content of the chewable portion by weight, and preferably less than 20%, for example 12%. Examples of flavourant include wood smoke, meat, liver powder, fish extracts, cheese, chocolate, and fermentation products such as yeast and malt. Flavourants may be included as a single entity or in combination.

Preferably the masticable portion comprises gelatin, edible gum (pref. arabic), polysaccharides (e.g. sugars such as sucrose and/or glucose, e.g. in the form of glucose syrup), hydrogenated polysaccharides (e.g. sorbitol, xylitol, etc.) and/or inverted sugars. More preferably the masticable portion consists of an admixture of these such as "Lycasin®", (which comprises hydrogenated derivatives of partially hydrolyzed starch, principally sorbitol and maltitol. The full specification for Lycasin® is described in Rockström, 1980 or from the commercial manufacturers: Roquette Frères (France) and Lycasin Starch Ltd (Sweden). A suitable grade of Lycasin® useful for the masticable portion is Lycasin 80/55®, which comprises mainly maltitol syrup. The full specification for Lycasin 80/55® is also described in Rockström, 1980.

The masticable portion may also contain natural and/or synthetic preservatives and/or antioxidants to modify the chemical and physical stability. When present preservatives and antioxidants will make up less than 5% of the total volume of the content, and preferably less than 1%. Examples of antioxidants include alpha-tocopherol, alkyl gallate derivatives, nordihydroguaiaretic acid, ascorbic acid, citric acid, sodium metabisuphate and sodium sulphite. Other antioxidants of interest include butylated hydroxy anisole (BHA) and butylated hydroxy toluene (BHT).

During processing, the ingredients of the masticable layer may need to be heated or otherwise processed further to ensure the correct gelatinous properties of this portion (see above for properties). The temperature needs to be carefully controlled in the cooking chamber, preferably with the use of a continuous cooking system operating under reduced pressure (a technique for cooking sugar mixtures which is well known in the art, Peters, 1989). The sources and models of typical industrial equipment to enable the processing are described in the pharmaceutical and confectionery literature, Peters, 1989.

The reservoir portion or portions comprises fluid, in the form of a solution or dispersion of a unit dose of the medicament either by itself (if suitable) or in a vehicle, with a viscosity of less than 800mPa.s at body temperature (~ 36-40°C), such that on mastication, the masticable portion is ruptured and the medicament can freely flow from the reservoir portion(s) into the oral cavity.
Preferably >10 % of the reservoir portion is released after 10s chewing by a typical dog, such as a beagle.

Preferably the reservoir vehicle is water or an aqueous fluid.

Material for the fluid-filled reservoir portion or portions may include the medicament itself or the medicament solubilised or dispersed in a vehicle with the aforementioned properties. The total reservoir portion(s) will make up less than 60% of the total dosage form by volume, and preferably less than 30%, for example 20%.
Examples of aqueous reservoir vehicles include polysaccharides and/or polyol solutions for example sorbitol, glucose, high maltose, glycerol, syrups or preferably a mixture of some of these, for example Lycasin® - preferably Lycasin 80/55®. The composition of these materials is as described above.

Suitable solutions of polymers may also be used to modify flow and viscosity characteristics, such as hydroxy propyl methyl cellulose (HPMC) solution, etc.. Examples of lipophilic (oillike) reservoir vehicles include polyethylene glycol (PEG), polypropylene glycol (PG), edible oils such as olive oil, sun-flower oil, hydrogenated castor oil, palm kernel oil, sesame oil, etc.

Some specific examples of materials, which can be used in the reservoir vehicles, include a number of products available from Karlshamns AB SE-374 82 Karlshamn, Sweden, and technical equivalents thereof, viz.:
(i) AKOMED R: Caprylic/capric triglyceride (CAS-No: 65381-09-1; 73398-61-5; EINECS-No: 265-724-3; 277-452-2);
(ii) AKOSOFT 36: Vegetable fat. Hard fat. (INCI name: Hydrogenated Coco-glycerides; CAS-No: 91744-42-2; EINECS-No: 294-604-3);
(iii) AKOSOL 403: Hydrogenated Palmkernel oil (CAS-No: 68990-82-9; EINECS-No: 273-627-2); and
(iv) AKOSOL 407: Hydrogenated Soybean oil (CAS-No: 8016-70-4; EINECS-No: 232-410-2).

The reservoir portion or portions may also contain natural and/or synthetic preservatives and/or antioxidants to modify the chemical and physical stability of the active agent contained therein. When present preservatives and antioxidants will make up less than 5% of the total volume of the content, and preferably lass than 1%. Examples of antioxidants include alpha-tocopherol, alkyl gallate derivatives, nordihydroguaiaretic acid, ascorbic acid, citric acid, sodium metabisuphate and sodium sulphite. Other antioxidants of interest include butylated hydroxy anisole (BHA) and butylated hydroxy toluene (BHT).

Flavourant and colourant may also be included in the reservoir portion, and when present will make up less than 30% of the total volume content, and preferably less than 10%. Examples of flavourant include wood smoke, meat, liver powder, fish extracts, cheese, chocolate, and fermentation products, such as yeast and malt.

Medicaments for local delivery to the oral cavity may include those pertinent to oral health such as those used to control/reduce plaque, to prevent/reduce halitosis, gingivitis, periodontitis and other periodontal infections.

Examples of these medicaments may include anti-bacterial agents, wherein the agents (their analogues and salts) are derived from bis-guanidino antibacterials such as chlorhexidine, hexetidine, alexidine, etc.; myxovirescin; cetyl pyridinium chloride; minocycline, doxycycline, chlortetracycline and other tetracycline antibacterials; anionic antibacterials such as triclosan etc.; nisin and other lantibiotics; malabaricone C and other argingipain inhibitors; ofloxacin and other quinolone antibacterials; sulfadiazine; actinobolin; histatins, bactenecin and other peptide antibacterials.

Further examples of these medicaments may include agents that have plaque anti-adherent properties, wherein the agents (their analogues and salts) are derived from morpholino-amino alcohols, such as delmopinol, octapinol etc., and other surface-active agents such as those derived from cationic and anionic surfactant classes.

Other examples of medicaments may also include antibacterial agents or antifungal agents used to treat infections of the oral cavity.

Other examples of medicaments may include agents that achieve systemic levels via sublingual or buccal absorption.

Other examples of medicaments may also include those used to prevent/treat systemic diseases such as antiparasitics, for example selamectin; antifungals, such as fluconazole and analogues; and others wherein the delivery route is via oral administration.

"Medicament" herein means a single active agent or a combination of active agents.

The invention provides scope for the delivery of medicaments in the solubilised or dispersed form in the reservoir portion of the dosage form either to the gastrointestinal tract or locally to the oral cavity. One aspect of the invention is particularly useful for maximising the lumenal exposure of lipophilic medicaments in animals (i.e., concentration within the gastrointestinal lumen), which is recognized to be limiting when the aqueous solubility of the medicament is low, typically less than 100µg/ml.

According to this invention, the ability to modify the nature of the reservoir vehicle, renders high flexibility in the choice of medicament which could be incorporated or solubilised into the dosage form as the release of the medicament occurs in the oral cavity. The medicament can be delivered in the oral cavity or along the gastrointestinal tract following ingestion.

Typical loading of the medicament in the reservoir portion can vary from 100%, where the medicament is in a suitable fluid form, to 0.001% (by weight/volume, or "w/v"), where the medicament is dispersed / solubilised. Where the medicament is dispersed and/or solubilised in the reservoir, the medicament typically forms 0.001 to 80%, preferably 0.01 to 20% w/v. Choice of medicament vehicle, concentration, etc. depends on factors such as the characteristics of the medicament, the size of the effect desired to achieve, the target subject group, etc.

The reservoir portion may contain further medicaments depending on the therapeutic need. When more than one medicament is present, the total drug loading in the reservoir portion is in the ranges mentioned above.

For oral health products, the speed of rupture and release of the medicament(s) from the reservoir portion is particularly pertinent. Typical rupture time to attain good coverage of the teeth, gum and tongue areas is in the range 1 to 30 seconds, preferably 1- 10 seconds. Preferably >10% of the reservoir portion is released after this time, more preferably >50%.

The total time for mastication once the active agent has been released is also crucial for oral health application or applications wherein effective distribution within the oral cavity is required. Typical total mastication time to attain good coverage on the teeth, gum and tongue areas is greater than 10 seconds, and preferably greater than 30 seconds.

Formulations of the invention are also especially useful for the oral delivery of medicaments that are poorly soluble, since the medicament can be solubilised in an appropriate vehicle in the reservoir portion and released along the gastrointestinal tract in a solubilised form. This negates the need for the medicament to undergo dissolution that may rate limit the overall oral absorption process. Medicaments that may benefit from this method of delivery include those with a partitioning coefficient (logD) >1 and aqueous solubility typically lower than 100µg/ml.

The dimensions of the dosage form disclosed in this invention can be specifically tailored to meet the particular needs of the application. Where the use is concerned with delivery of the medicament to the oral cavity, the dimensions should be such that the dosage form cannot be readily swallowed before mastication. Thus, whilst it is recognised that the dimensions required to induce mastication is species size and variety dependent, the observation specific to this invention is that the dosage form should be typically above 10% of the volume of the oral cavity of that particular animal species.

The volume as described in the above context refers to the interior portion of the oral cavity enclosed within the jaw-line. This volume can be estimated using photographic methods or direct physical measurements of the jaw-line geometry.

In applications wherein local oral exposure is not pertinent, then there is no preferred range on the overall dimensions of the dosage form.

The dosage form as described in this invention can be of any shape as long as the entire dosage form can be accommodated into the oral cavity in a single administration. For processing reasons the preferred shapes include substantially hemispherical, cubic and ovoid. These configurations can be designed to optimise the ratio of the reservoir portion to that of the overall dosage form by volume.

A preferred dosage form suitable for oral health care for domestic animals, e.g. dogs and cats, comprises the following (the percentages expressed are that of the total solid content of the relevant portion by weight):
a masticable portion containing gelatine (about 20%), gum arabic (about 3%) and flavourants:
pork liver powder and beef powder, each at about 6%; in a hydrogenated polysaccharide Lycasin® base;
a reservoir portion comprising Lycasin®, malt flavouring (about 1%) by weight and the medicament (for example an oral health agent such as delmopinol at up to about 3% by weight).

A preferred dosage form shape is substantially hemispherical.
Preferably the reservoir portion will represent a maximum of 40% by volume of the final dosage form, more preferably 5 - 25% by volume.

The dosage forms, as described herein, can be prepared by any suitable process known to those skilled in such arts, including by a co-extrusion and depositing process wherein the masticable portion and reservoir portion are prepared separately and co-extruded to form a unit dose. The masticable portion forms the outer casing, which encapsulates the inner reservoir portion. Unit doses of the co-extruded mass are deposited accurately into a starch mould and dried over a period of ca. 24 to ca. 72 hours to allow the outer masticable portion to set. Alternatively, continuous extrusion processes may also be used, wherein the masticable portion and the reservoir portion are extruded as wet-masses into a continuous "rope", and rendered to the appropriate size by cutting the rope to yield unit dosage forms which are then dried in a conventional convection oven (Peters, 1989). Extrusion of continuous feed based on the well-known Buhler method may also be used.

The performance of the formulation, especially as related to local oral delivery, may be determined by measuring the level of exposure of the reservoir portion, pre-incorporated with a blue food dye, to quantify coverage within the oral cavity. Where the dosage form is chewed or gnawed by the animal then extensive coverage of the teeth, gum and tongue areas within the oral cavity may be achieved. This is particularly useful for application in oral health where the medicament is required to be distributed widely within the oral cavity for therapeutic efficacy.

We have found that the overall size of the dosage form is a pertinent parameter for promoting prolonged chewing and extended residence time in the oral cavity. Where the volume of the dosage form exceeds a critical percentage of the total volume of the animal's oral cavity, then the subject is forced to chew to render the dosage form into smaller-sized portions which can be swallowed without causing obstructions. Typically the dosage form volume is about 5-50% of that of the animal's oral cavity.

It is envisaged that a range of formulation sizes / shapes can be produced, adapted to the size and / or breed of animal concerned, as well as their therapeutic need. The formulation volume ranges, based on body weight, of a typical companion animal, such as a dog, is shown below in table 1.

**Table 1**

| **Dog body weight (kg)** | **Typical volume of formulation (cm**^{**3**}**)** |
|---|---|
| Up to 2 | Up to 5 |
| 2.1-5 | 2 to 10 |
| 5.1 to 10 | 5 to 15 |
| 10.1 to 20 | 10 to 30 |
| 20.1 to 40 | 20 to 50 |

The invention is illustrated by the following examples.

Table 2 shows the level of coverage of the material emanating from the reservoir portion (using a blue dye) within the oral cavity following administration of the dosage form provided in Example 1.

The components of the formulation as shown in the formula according to Example 1, are expressed in terms of the percent total solid (TS) which forms the outer masticable portion, the percent by weight of the total dosage form and the mass per unit of dosage form. This method of formula expression is well known in art for liquid-filled dosage forms (Peters, 1989).

### Example 1: Fluid-filled dosage form (sugar-based)

| **Components** | **% Total solid (TS)**^{**1**} **content by weight** | **% Total dosage form by weight** | **g/unit** |
|---|---|---|---|
| **Outer portion** | | | |
| Sucrose | 46.35 | 29.80 | 7.233 |
| Glucose syrup | 27.65 | 17.78 | 4.315 |
| Gelatin | 14.00 | 9.00 | 2.184 |
| Pork liver powder | 6.00 | 3.86 | 0.936 |
| Yeast extract | 6.00 | 3.86 | 0.936 |
| Water | N/A | 18.12 | 4.396 |
| | | | |

| **Reservoir portion** | | | |
|---|---|---|---|
| Lycasin 80/55® | N/A | 17.23 | 4.180 |
| Blue dye | N/A | 0.34 | 0.084 |
| | **Total** | **100** | **24.264** |

| | | | |
|---|---|---|---|
| ¹TS - Total solid content of the outer portion of the dosage form | | | |

The gelatin was dissolved in 22.5ml of water in an appropriate glass vessel (e.g. 100ml Pyrex glass beaker), covered and then placed in a water bath at 55°C until a homogenous clear, yellow viscous solution was produced. The flavourants (pork liver powder and yeast extract) were then added to the gelatin solution, thoroughly mixed and stored at 55°C until required.

The sucrose and glucose syrup were weighed into a suitable cooking vessel (e.g., a 2-litre Teflon coated pan), added with the remaining water and heated to between 115-130°C, preferably 123°C, with constant, gentle stirring and regular temperature monitoring. Once the target temperature was obtained, the cooking vessel was removed from the heat and the mixture cooled to ~ 90°C, with continuous stirring and regular temperature monitoring. The gelatin-flavour solution was then added and thoroughly stirred into the mixture to produce a homogenous, viscous vehicle mixture. This vehicle mixture was then poured into the hopper of the co-depositor (Center in shell laboratory depositor PLC controlled, Supplied by Werner Makat GmbH & Co.) designated for the outer shell. The hopper designated for the inner fluid-vehicle, was filled with the Lycasin® solution added with the blue dye, and maintained at 55°C.

The co-depositor was then used to deposit 16mL from the hopper designated for the outer shell, and 4mL from the hopper designated for the inner fluid-vehicle. The co-extruded mass was then left to set in pre-formed hemispherical starch impression moulds, constructed by imprinting solid wooden hemispherical blocks into the starch spaced 10cm apart, each corresponding to a volume of 20mL. Once the masses had set in the mould (up to 72 hours), they were removed from the starch bed, dusted, wrapped in greaseproof paper and stored in airtight plastic securitainers.

A formulation according to Example 1 was administered to a set of 8 dogs weighing in the range of 12-18kg and was chewed for greater than 20 seconds. At the end of this period the formulation was swallowed and the dog's oral cavity examined. The extent of coverage of the blue dye represents the area was as follows: -

**Table 2**

| **Oral Tissues** | **Mean Percentage Coverage** (by area as measured visually and by still digital photography) |
|---|---|
| premolar/molar teeth | 80 |
| upper gum margin | 60 |
| lower gum margin | 60 |
| incisors/canine teeth | 50 |
| tongue surface | 95 |
| throat | <5 |

This indicates that the dye was extensively distributed to all the areas within the oral cavity that are important targets for oral health, demonstrating the efficiency of delivery of reservoir material to the oral cavity.

### Example 2: Fluid-filled dosage form (sugar-free base)

| **Components** | **% Total solid (TS)**^{**1**} **content by weight** | **% Total dosage form by weight** | **g/unit** |
|---|---|---|---|
| **Outer portion** | | | |
| Lycasin 80/55 | 65.00 | 41.74 | 10.143 |
| Gelatin | 20.00 | 12.84 | 3.121 |
| Gum arabic | 3.00 | 1.93 | 0.468 |
| Pork liver powder | 6.00 | 3.85 | 0.936 |
| Beefpowder | 6.00 | 3.85 | 0.936 |
| Water | N/A | 18.09 | 4.396 |
| | | | |

| **Reservoir portion** | | | |
|---|---|---|---|
| Lycasin 80/55® | N/A | 17.04 | 4.140 |
| Malt | N/A | 0.17 | 0.040 |
| Delmopinol | N/A | 0.49 | 0.120 |
| | **Total** | **100** | **24.300** |

| | | | |
|---|---|---|---|
| ¹TS - Total solid content of the outer portion of the dosage form | | | |

The formulation provided in the above example can be prepared using the method described previously in Example 1, replacing the sucrose and glucose syrup with Lycasin 80/55® and the dye with the medicament delmopinol.

Further Examples are made in the same way as described above for Example 1 and contain the following components:

### Masticable outer portion composition:

| **Identity** | **Gelatin (%TS)** | **Gum Arabic (%TS)** | **Pectin (%TS)** | **Lycasin 80/55® (%TS)** | **Water content** |
|---|---|---|---|---|---|
| A | 14.5 | 1.0 | 0.1 | 50.6 | 21.8 |
| B | 2.0 | 5.0 | 0.1 | 66.9 | 14.0 |
| C | 4.75 | 1.0 | 1.0 | 67.25 | 14.0 |
| D | 12.0 | 5.0 | 1.0 | 48.2 | 21.8 |
| E | 12.5 | 1.0 | 0.1 | 52.6 | 21.8 |
| F | 4.0 | 5.0 | 0.1 | 64.9 | 14.0 |
| G | 6.75 | 1.0 | 1.0 | 65.25 | 14.0 |
| H | 10.0 | 5.0 | 1.0 | 50.2 | 21.8 |
| I | 4.75 | 1.0 | 0.1 | 68.15 | 14.0 |
| J | 12.0 | 5.0 | 0.1 | 49.1 | 21.8 |
| K | 14.5 | 1.0 | 1.0 | 49.7 | 21.8 |
| L | 2.0 | 5.0 | 1.0 | 66.0 | 14.0 |
| M | 6.75 | 1.0 | 0.1 | 66.15 | 14.0 |
| N | 10.0 | 5.0 | 0.1 | 51.1 | 21.8 |
| O | 12.5 | 1.0 | 1.0 | 51.7 | 21.8 |
| P | 4.0 | 5.0 | 1.0 | 64.0 | 14.0 |
| Q | 12.5 | 3.0 | 0.5 | 50.2 | 21.8 |

The outer masticable portion also contains flavouring (e.g. pork liver flavouring) at 12%TS

### Reservoir Portion

### (A) Vehicle fills:

1 Lycasin 80/55®
2 Glucose syrup
3 PEG 300
4 PEG 600

The reservoir also contains malt flavouring up to 0.8%.

### (B) Drug concentrations:

delmopinol at 1%w/v, 2%w/v and 3%w/v
octapinol at 1%w/v, 2%w/v and 3%w/v

Plaque index measurements were measured by a modification of the well-known methods of Silness and Loe (Silness, J. and Löe, H. (1964) Periodontal disease in pregnancy. II. Correlation between oral hygiene and periodontal condition. *Acta Odontol. Scand*. **22,** 121-135; Löe, H (1967) The gingival index, the plaque index and the retention index systems. *J*. *Periodontol*. **38,** 610-616.) The modification that we made to it is that we assess the plaque at only 3 points along the gingival margin, instead of the 4 sites described in the original method.

Variations to produce more Examples are made by:
using the same masticable layer components mentioned above, substituting the flavouring for another flavour, such as will be acceptable to whichever species of animal is to be treated;
using the same reservoir components mentioned above, substituting the flavouring for another flavour, such as will be acceptable to whichever species of animal is to be treated;
using the same reservoir components mentioned above, substituting the medicament for
another medicament or medicaments, such as are suitable for the treatment of the specific condition to be treated, such as those specifically mentioned above, and to whichever species, size, etc. of animal is to be treated (including variation of the dosages as appropriate); and
variation of the size of the final formulation to suit the size of oral cavity of whichever species is to be treated;

The formulations described above may be deposited into any shapes, sizes etc., pre-formed in starch moulds as described above.

It is of course to be recognised that multitudinous other variations of the Examples mentioned herein are included within the spirit of the invention.

All references mentioned herein are incorporated in their entirety.

Where "treatment" is mentioned, it is to be understood as including preventative treatment as well as alleviation and cure of medical conditions.

### References

Ashby, M.F. and Jones, D.R.H. (1980) *Engineering Materials 1 - An introduction to the their properties and applications*, Pergamon Press, Exeter, pp. 29-35 Jones, D.S., Woolfson, A.D. and Djokic, J. (1996) Texture profile analysis of bioadhesive polymeric semisolids: mechanical characterization and investigation of interactions between formulation components. *J. Appl*. *Polym*. *Sci.* **61,** 2229-2234.
Jones, D.S. (1999) Dynamic mechanical analysis of polymeric systems of pharmaceutical and biomedical significance. *Int*. *J*. *Pharm*. **179,** 167-178.
Peters, D. (1989) in Pharmaceutical Dosage forms: Tablets, Ed. Liberman, H., Lachman, L., and Schwartz, J., Marcel Dekker, New York, pp. 497-500
Rockström, E. (1980). Lycasin hydrogenated hydrosylates, in carbohydrates sweeteners in food and nutrition, Academic Press, New York, pp. 225-232
Silness, J. and Löe, H. (1964) Periodontal disease in pregnancy. II. Correlation between oral hygiene and periodontal condition. *Acta Odontol*. *Scand*. **22,** 121-135
Löe, H (1967) The gingival index, the plaque index and the retention index systems. *J. Periodontol*. **38,** 610-616

## Claims

1. An oral formulation for a medicament comprising:
a solid masticable portion and one or more reservoir portions encompassed by said solid masticable portion;
the solid masticable portion consisting of a fully edible material, having a Young's modulus of
0.01-5MPa, and compressive strength in the range 10-10,000 mJ,
the reservoir portion or portions comprising a releasable dose of the medicament in a fluid (preferably a liquid) form, with a viscosity below 800mPa.s at body temperature (ca. 36-ca.40°C),
such that on mastication, the masticable portion is ruptured and the unit dose of the medicament is released in a short space of time from the reservoir portion into the oral cavity.

2. A formulation according to claim 1 where the viscosity of reservoir portion is in the range 50-200 mPa.s.

3. A formulation according to claim 1 which consists of:
a masticable portion containing gelatine (about 20%), gum arabic (about 3%),
pork liver powder and/or beef powder as flavourant, each at about 6%,
in a hydrogenated polysaccharide Lycasin® base;
a reservoir portion comprising Lycasin®, malt flavouring (about 1%) by weight and the medicament.

4. A formulation according to any previous claim wherein the reservoir portion is up to 40% by volume of the formulation.

5. A formulation according to claim 4 wherein the reservoir portion is 5 - 25% by volume.

6. A formulation according to any previous claim wherein the shape of the formulation is substantially hemispherical.

7. A formulation according to claim 1 wherein the masticable portion contains gelatine (at about 20%), gum arabic (at about 3%) and flavourants: pork liver powder and beef powder, each at about 6%; in a hydrogenated polysaccharide Lycasin® base;
and the reservoir portion comprises Lycasin®, malt flavouring (at about 1%) by weight and the medicament.

8. A formulation according to any previous claim wherein the medicament is present at up to about 3% by weight.

9. A formulation according to any previous claim wherein the medicament is one that reduces plaque, controls gingivitis, prevents calculus, prevents halitosis or prevents periodontitis.

10. A formulation according to any one of claims 1 to 8 wherein the medicament is delmopinol or octapinol.

11. A method of treatment of a human or non-human animal subject in need of treatment with a medicament comprising administration of a formulation of said medicament according to any previous claim.
